# EUROPEAN PATENT APPLICATION

(11) **EP 4 501 275 A1**
(43) Date of publication of application: **05.02.2025**
(21) Application number: 22940848.9
(22) Date of filing: 12.05.2022
(51) Int. Cl.: A61C 13/00, A61C 9/00, G16H 50/50, A61B 5/00, G06F 17/00, A61B 18/20

(54) **METHOD FOR AUTOMATICALLY ALIGNING THREE-DIMENSIONAL SINGLE TOOTH MODEL ON THREE-DIMENSIONAL ORAL SCAN DATA, AND COMPUTER-READABLE RECORDING MEDIUM IN WHICH PROGRAM FOR EXECUTING SAME ON COMPUTER IS RECORDED**

(30) Priority: 04.05.2022 KR 20220055252
(71) Applicant: Imagoworks Inc., Seoul 02455 (KR)
(72) Inventor: SHIN, Bonjour, Seoul 05379 (KR); KIM, Hannah, Seoul 05379 (KR); LEE, Taeseok, Seongnam-si Gyeonggi-do 13611 (KR); KAM, Dong Uk, Seoul 08832 (KR); CHOI, Jinhyeok, Seoul 06295 (KR); SON, Tae-geun, Seoul 08761 (KR); KIM, Youngjun, Seoul 06610 (KR)
(74) Representative: BCKIP Part mbB
(86) International application number: PCT/KR2022/006842
(87) International publication number: WO 2023/214607

(57) **Abstract**

An automated method for aligning a 3D single tooth model to 3D oral scan data includes determining an oral scan landmark and a teeth curve formed by teeth in the 3D oral scan data, determining a margin line of a target tooth in the 3D oral scan data and a region of interest in the 3D oral scan data, determining first to third axes of the 3D oral scan data in the region of interest based on the oral scan landmark, determining a single tooth landmark of the 3D single tooth model, determining fourth to sixth axes of the 3D single tooth model based on the single tooth landmark and aligning the 3D single tooth model to the 3D oral scan data such that the fourth to sixth axes of the 3D single tooth model respectively overlap the first to third axes of the 3D oral scan data.

## Description

### [TECHNICAL FIELD]

The present inventive concept relates to an automated method for aligning a three dimensional ("3D") single tooth model to 3D oral scan data and a non-transitory computer-readable storage medium having stored thereon program instructions of the automated method for aligning the 3D single tooth model to the 3D oral scan data. More particularly, the present inventive concept relates to an automated method for aligning a 3D single tooth model to 3D oral scan data to reduce time and process of manufacturing prostheses and a non-transitory computer-readable storage medium having stored thereon program instructions of the automated method for aligning the 3D single tooth model to the 3D oral scan data.

### [BACKGROUND]

3D oral scan data may refer to data scanned by a 3D scanner for teeth and oral cavity, an impression model of the teeth and the oral cavity or a reconstruction model of the teeth and the oral cavity. In dental treatment such as prosthetic treatment including in-ray, on-ray and crown, implant and orthodontics oral data of a patient may be obtained and may be used to design prostheses or implants and manufacture braces.

Conventionally, a method of manufacturing prostheses, implants, braces by hand after directly modeling the oral cavity using alginate or the like has been mainly used. Recently, a digital method including obtaining 3D oral scan data of a patient using the 3D scanner, designing prostheses, implants and braces using a computer, and 3D printing them has been gradually used.

In the digital method, a 3D single tooth model which is a dental library model predesigned to some extent for each tooth type (tooth number) or mesh data generated by individuals such as dental technicians and dentists may be used.

In order to digitally manufacture prostheses, implants and braces, the 3D single tooth model may be aligned to the 3D oral scan data. If the process of aligning the 3D single tooth model to the 3D oral scan data is performed manually, a work fatigue of the dentist or dental technician may increase and an accuracy and a productivity of the result may decrease.

### [DETAILED EXPLANATION OF THE INVENTION]

### [TECHNICAL PURPOSE]

The purpose of the present inventive concept is providing an automated method for aligning a 3D single tooth model to 3D oral scan data to reduce time and process of manufacturing prostheses, implants, braces and dental instruments.

Another purpose of the present inventive concept is providing a non-transitory computer-readable storage medium having stored thereon program instructions of the automated method for aligning the 3D single tooth model to the 3D oral scan data.

### [TECHNICAL SOLUTION]

In an example automated method for aligning a three dimensional ("3D") single tooth model to 3D oral scan data according to the present inventive concept, the method includes determining an oral scan landmark of the 3D oral scan data and a teeth curve formed by teeth in the 3D oral scan data, determining a margin line of a target tooth in the 3D oral scan data and a region of interest in the 3D oral scan data, determining a first axis, a second axis and a third axis of the 3D oral scan data in the region of interest based on the oral scan landmark, determining a single tooth landmark of the 3D single tooth model, determining a fourth axis, a fifth axis and a sixth axis of the 3D single tooth model based on the single tooth landmark and aligning the 3D single tooth model to the 3D oral scan data such that the fourth axis, the fifth axis and the sixth axis of the 3D single tooth model respectively overlap the first axis, the second axis and the third axis of the 3D oral scan data.

In an embodiment of the present inventive concept, the method may further include scaling the 3D single tooth model based on the margin line of the target tooth and adjacent teeth adjacent to the target tooth of the 3D oral scan data.

In an embodiment of the present inventive concept, the method may further include adjusting a height of the 3D single tooth model based on a height of an adjacent tooth adjacent to the target tooth in the 3D oral scan data.

In an embodiment of the present inventive concept, the method may further include adjusting a width of the 3D single tooth model based on a contact point where the 3D single tooth model contacts an adjacent tooth adjacent to the target tooth in the 3D oral scan data.

In an embodiment of the present inventive concept, the first axis of the 3D oral scan data may be defined by a tangential direction of the teeth curve at the target tooth.

In an embodiment of the present inventive concept, the second axis of the 3D oral scan data may represent an insertion direction of a tooth or an opposite direction of the insertion direction.

In an embodiment of the present inventive concept, the third axis of the 3D oral scan data may be defined by a cross product of a first vector of the first axis and a second vector of the second axis.

In an embodiment of the present inventive concept, the oral scan landmark may include at least three landmarks disposed in the 3D oral scan data.

In an embodiment of the present inventive concept, the oral scan landmark may include a first landmark disposed at a first end portion of the teeth curve, a second landmark disposed at a second end portion of the teeth curve and a third landmark disposed at a central point of the teeth curve.

In an embodiment of the present inventive concept, the fourth axis of the 3D single tooth model may represent a horizontal direction of the tooth in the 3D single tooth model.

In an embodiment of the present inventive concept, the fifth axis of the 3D single tooth model may represent a direction toward an occlusal surface of the tooth in the 3D single tooth model or an opposite direction of the direction toward the occlusal surface.

In an embodiment of the present inventive concept, the sixth axis of the 3D single tooth model may be defined by a cross product of a fourth vector of the fourth axis and a fifth vector of the fifth axis.

In an embodiment of the present inventive concept, the single tooth landmark may include at least two landmarks defined on a tooth in the 3D single tooth model to determine the fourth axis.

In an embodiment of the present inventive concept, the single tooth landmark may include at least two landmarks defined on the tooth in the 3D single tooth model to determine the fifth axis.

In an embodiment of the present inventive concept, the oral scan landmark and the teeth curve may be determined using a first artificial intelligence neural network.

In an embodiment of the present inventive concept, the margin line of the target tooth in the 3D oral scan data and the region of interest may be determined using a second artificial intelligence neural network different from the first artificial intelligence neural network.

In an embodiment of the present inventive concept, the single tooth landmark of the 3D single tooth model may be determined using a third artificial intelligence neural network different from the first artificial intelligence neural network and the second artificial intelligence neural network.

In an embodiment of the present inventive concept, the method may further include adjusting a height of the 3D single tooth model based on a first distance from a first plane in the 3D oral scan data to a first point of the target tooth in the 3D oral scan data and a second distance from the first plane to a second point of the 3D single tooth model.

In an embodiment of the present inventive concept, the first distance may represent a distance from the first plane to a furthest position of the target tooth. The second distance may represent a distance from the first plane to a closest position of an occlusal surface of the 3D single tooth model. The height of the 3D single tooth model may be adjusted such that the second distance matches the first distance.

In an embodiment of the present inventive concept, a program for executing the automated method for aligning the 3D single tooth model to the 3D oral scan data on a computer may be recorded on a computer-readable recording medium.

### [EFFECT OF THE INVENTION]

According to the automated method for aligning the 3D single tooth model to the 3D oral scan data, the process of aligning the 3D single tooth model to the 3D oral scan data is performed automatically so that a work fatigue of the dentist or dental technician may decrease and an accuracy of the aligning result may increase.

In addition, the aligned single tooth model may be used to manufacture prostheses, implants, braces and dental instruments so that an effort and a time of manufacturing the prostheses, the implants, the braces and the dental instruments may decrease and an accuracy and a productivity of the prostheses, the implants, the braces and the dental instruments may increase.

In addition, a deep learning may be used in some steps of the automated method for aligning the 3D single tooth model to the 3D oral scan data. When the deep learning is be used in some steps, the work fatigue of the dentist or dental technician may further decrease and the accuracy of the aligning result may further increase.

### [BRIEF EXPLANATION OF THE DRAWINGS]

FIG. 1 is a flowchart diagram illustrating an automated method for aligning a 3D single tooth model to 3D oral scan data according to an embodiment of the present inventive concept.
FIG. 2a is a perspective view illustrating an example of the 3D oral scan data.
FIG. 2b is a perspective view illustrating an example of the 3D oral scan data.
FIG. 2c is a perspective view illustrating an example of the 3D single tooth model.
FIG. 2d is a perspective view illustrating an example of the 3D single tooth model.
FIG. 2e is a perspective view illustrating an example of the 3D single tooth model.
FIG. 2f is a perspective view illustrating an example of the 3D single tooth model.
FIG. 3 is a diagram illustrating a 3D oral scan data landmark and a teeth curve of FIG. 1.
FIG. 4 is a diagram illustrating a margin line of the 3D oral scan data of FIG. 1.
FIG. 5 is a diagram illustrating a first axis, a second axis and a third axis of the 3D oral scan data of FIG. 1.
FIG. 6 is a diagram illustrating a fourth axis, a fifth axis and a sixth axis of the 3D single tooth model of FIG. 1.
FIG. 7 is a diagram illustrating a lower surface of the 3D single tooth model of FIG. 1 corresponding to the margin line of the 3D oral scan data of FIG. 1.
FIG. 8 is a diagram illustrating an example of scaling the 3D single tooth model of FIG. 1 based on the margin line of the 3D oral scan data of FIG. 1.
FIGS. 9 and 10 are diagrams illustrating examples of scaling the 3D single tooth model of FIG. 1.
FIG. 11a is a diagram illustrating an example of 3D oral scan data and FIG. 11b is a diagram illustrating a result of combining a 3D single tooth model to the 3D oral scan data of FIG. 11a.
FIG. 12a is a diagram illustrating an example of 3D oral scan data and FIG. 12b is a diagram illustrating a result of combining a 3D single tooth model to the 3D oral scan data of FIG. 12a.
FIG. 13a is a diagram illustrating an example of 3D oral scan data and FIG. 13b is a diagram illustrating a result of combining a 3D single tooth model to the 3D oral scan data of FIG. 13a.

### [BEST MODE FOR CARRYING OUT THE INVENTION]

The present inventive concept now will be described more fully hereinafter with reference to the accompanying drawings, in which exemplary embodiments of the present invention are shown. The present inventive concept may, however, be embodied in many different forms and should not be construed as limited to the exemplary embodiments set forth herein.

Rather, these exemplary embodiments are provided so that this disclosure will be thorough and complete, and will fully convey the scope of the present invention to those skilled in the art.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention.

It will be understood that when an element or layer is referred to as being "connected to" or "coupled to" another element or layer, it can be directly connected or coupled to the other element or layer or intervening elements or layers may be present. In contrast, when it is referred that an element is "directly connected to" or "directly coupled to" another element or layer, there are no intervening elements or layers present. Other expressions describing the relationship between elements, such as "between" and "directly between" or "adjacent to" and "directly adjacent to", etc., should be interpreted similarly. Like numerals refer to like elements throughout. As used herein, the term "and/or" includes any and all combinations of one or more of the associated listed items.

The terminology used herein is for the purpose of describing particular exemplary embodiments only and is not intended to be limiting of the present invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the inventive concept as used herein.

Hereinafter, preferred embodiments of the present inventive concept will be explained in detail with reference to the accompanying drawings. The same reference numerals are used for the same elements in the drawings, and duplicate explanations for the same elements may be omitted.

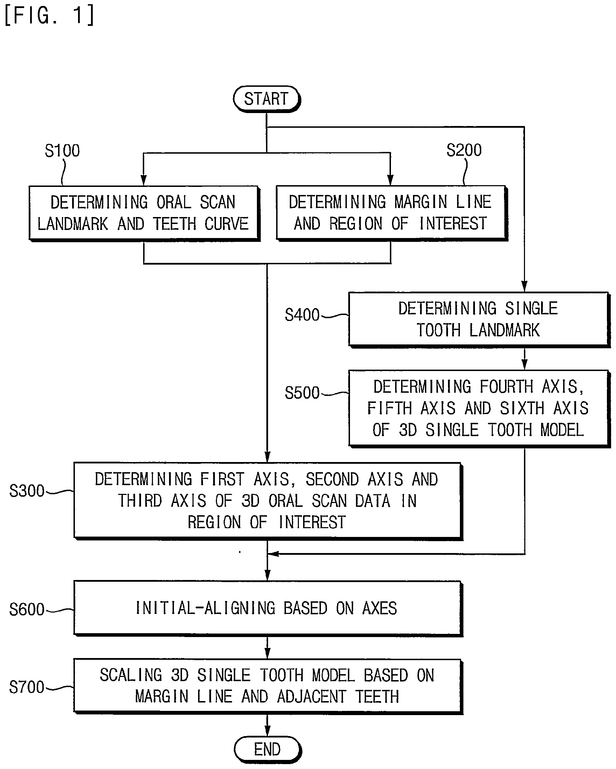

FIG. 1 is a flowchart diagram illustrating an automated method for aligning a 3D single tooth model to 3D oral scan data according to an embodiment of the present inventive concept.

Referring to FIG. 1, an automated method for aligning the 3D single tooth model to 3D oral scan data includes determining an oral scan landmark of the 3D oral scan data and a teeth curve formed by teeth in the 3D oral scan data (step S100), determining a margin line of a target tooth in the 3D oral scan data and a region of interest (step S200), determining a first axis, a second axis and a third axis of the 3D oral scan data in the region of interest based on the oral scan landmark (step S300), determining a single tooth landmark of the 3D single tooth model (step S400), determining a fourth axis, a fifth axis and a sixth axis of the 3D single tooth model based on the single tooth landmark (step S500) and aligning the 3D single tooth model to the 3D oral scan data such that the fourth axis, the fifth axis and the sixth axis of the 3D single tooth model respectively overlap the first axis, the second axis and the third axis of the 3D oral scan data (step S600). In addition, the automated method for aligning the 3D single tooth model to the 3D oral scan data may further include scaling the 3D single tooth model based on the margin line of the target tooth and adjacent teeth adjacent to the target tooth of the 3D oral scan data (step S700).

The automated method for aligning the 3D single tooth model to the 3D oral scan data of the present embodiment may be operated by a computing apparatus.

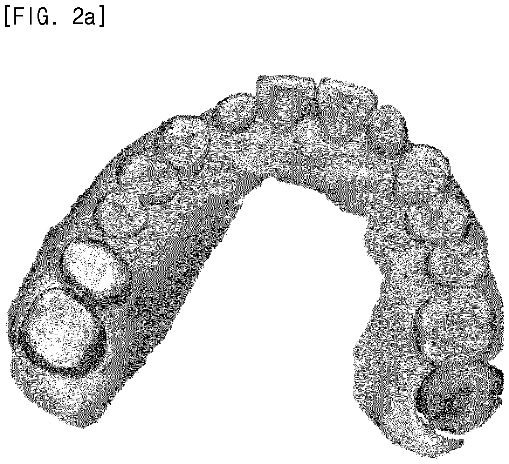

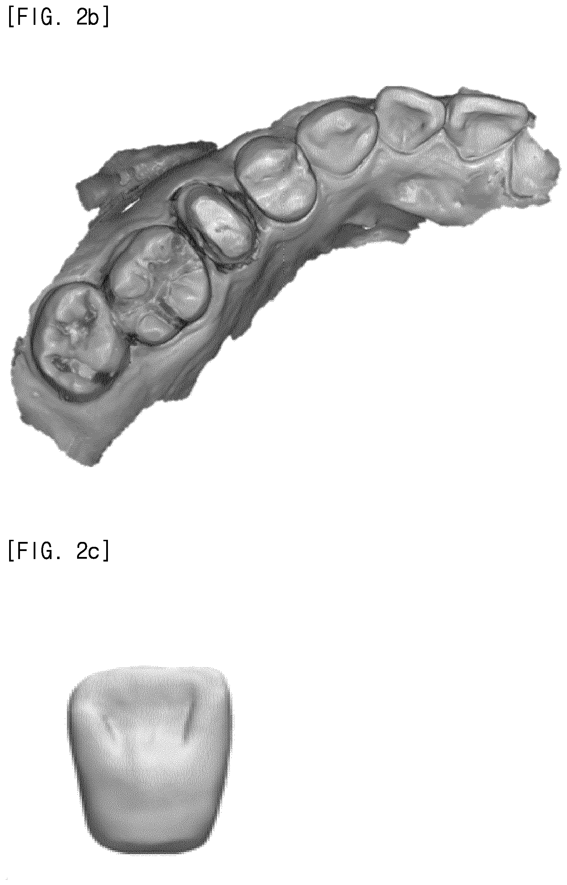

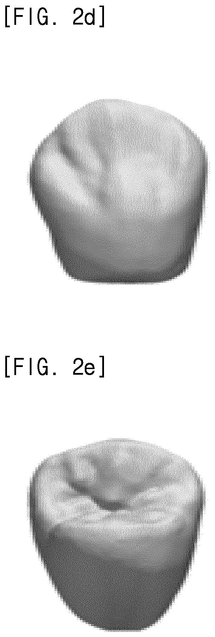

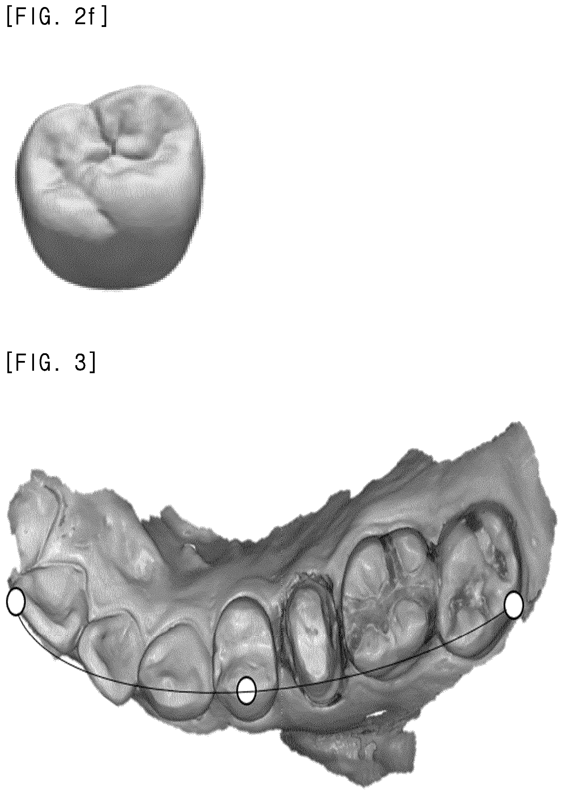

FIG. 2a is a perspective view illustrating an example of the 3D oral scan data. FIG. 2b is a perspective view illustrating an example of the 3D oral scan data. FIG. 2c is a perspective view illustrating an example of the 3D single tooth model. FIG. 2d is a perspective view illustrating an example of the 3D single tooth model. FIG. 2e is a perspective view illustrating an example of the 3D single tooth model. FIG. 2f is a perspective view illustrating an example of the 3D single tooth model.

The 3D oral scan data may refer to data scanned by a 3D scanner for teeth and oral cavity, an impression model of the teeth and the oral cavity or a reconstruction model of the teeth and the oral cavity. For example, the 3D oral scan data may be mesh data including 3D vertexes and triangles or rectangles generated by connecting the 3D vertexes. The 3D oral scan data may be image data captured by the 3D scanner. A filename extension of the 3D oral scan data is not limited, and may be, for example, one of ply, obj and stl.

FIGS. 2a and 2b represent examples of the 3D oral scan data. For example, the 3D oral scan data of FIG. 2a may be oral scan data for a full arch of a mandible or a full arch of a maxilla. For example, the 3D oral scan data of FIG. 2b may be oral scan data for a partial arch of the mandible or a partial arch of the maxilla. The automated method for aligning the 3D single tooth model to the 3D oral scan data of the present embodiment may be applied to both of the 3D oral scan data of the full arch as shown in FIG. 2a and the 3D oral scan data of the partial arch as shown in FIG. 2b.

The 3D single tooth model may be a dental library model predesigned to some extent for each tooth type (tooth number) or mesh data generated by individuals such as dental technicians and dentists.

The dental library model is a kind of sample tooth (a standard tooth) used to manufacture the prostheses, the implants and the braces and may have a typical tooth shape. The dental library model may have one sample tooth (the standard tooth) for each tooth number. The 3D oral scan data are captured by the 3D scanner so that the 3D oral scan data may have a low degree of completion of the mesh. When the degree of the completion of the mesh is low, a 3D printing may be inappropriate for manufacturing the prostheses, the implants and the braces. In contrast, the 3D dental library model may have a relatively high degree of completion of the mesh. Thus, when the prostheses, the implants and the braces are manufactured by deforming the 3D dental library model, the 3D printing may be very suitable for manufacturing the prostheses, the implants and the braces. Accordingly, when the 3D dental library model is aligned with the patient's oral scan data, the 3D dental library model aligned with the oral scan data may be an intermediate model suitable for digitally manufacturing prostheses, implants and braces.

FIGS. 2c, 2d, 2e and 2f represent examples of the 3D single tooth model. For example, FIG. 2c may represent a 3D single tooth model of a front tooth (an incisor). For example, FIG. 2d may represent a 3D single tooth model of a canine. For example, FIG. 2e may represent a 3D single tooth model of a premolar. For example, FIG. 2f may represent a 3D single tooth model of a molar. The automated method for aligning the 3D single tooth model to the 3D oral scan data of the present embodiment may be applied to all of the 3D single tooth model of the incisor in FIG. 2c, the 3D single tooth model of the canine in FIG. 2d, the 3D single tooth model of the premolar in FIG. 2e and the 3D single tooth model of the molar in FIG. 2f.

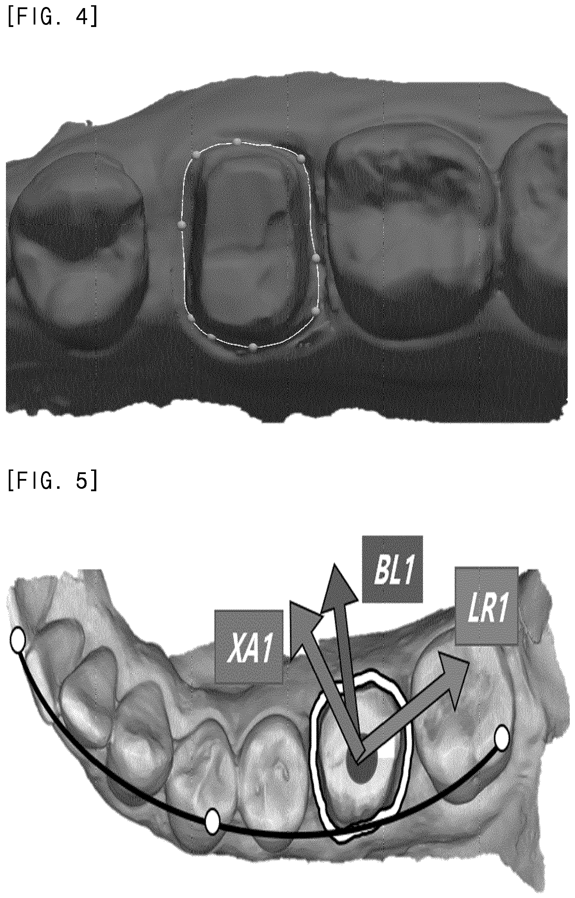

FIG. 3 is a diagram illustrating a 3D oral scan data landmark and a teeth curve of FIG. 1. FIG. 4 is a diagram illustrating a margin line of the 3D oral scan data of FIG. 1. FIG. 5 is a diagram illustrating a first axis LR1, a second axis XA1 and a third axis BL1 of the 3D oral scan data of FIG. 1.

Referring to FIGS. 1 to 5, the oral scan landmark of the 3D oral scan data and the teeth curve formed by teeth in the 3D oral scan data may be determined (step S100). FIG. 3 represents an example of the oral scan landmark and the teeth curve.

The step (step S100) of determining the oral scan data of the 3D oral scan data and the teeth curve may be manually operated by a user or be automatically operated by a deep learning. For example, the oral scan landmark and the teeth curve may be determined using a first artificial intelligence neural network.

The oral scan landmark may include at least three landmarks disposed in the 3D oral scan data. For example, the oral scan landmark may be disposed on teeth of the 3D oral scan data.

For example, the oral scan landmark may include a first landmark disposed at a first end portion of the teeth curve formed by the teeth in the 3D oral scan data, a second landmark disposed at a second end portion of the teeth curve and a third landmark disposed at a central point of the teeth curve.

For example, the oral scan landmark may include a first landmark disposed at a last tooth of a first end in a horizontal direction of the 3D oral scan data, a second landmark disposed at a last tooth of a second end in the horizontal direction of the 3D oral scan data and a third landmark representing a center of two central incisors of the 3D oral scan data.

The margin line of the target tooth and the region of interest may be determined in the 3D oral scan data. FIG. 4 represents an example of the margin line and the region of interest.

In FIG. 4, the target tooth at which the 3D single tooth model is aligned may be a prepared tooth (a prepped tooth). The prepared tooth may mean a tooth prepared for a crown. The prepared tooth may mean a tooth from which portion of the tooth has been shaped.

In FIG. 4, the margin line of the target tooth may be determined and an area within a closed curve formed by the margin line may be defined as the region of interest. For example, the margin line may be determined based on an area of the prepared tooth. Alternatively, the margin line may be determined by predicting an area of the tooth in an unprepared tooth state.

The step (step S100) of determining the margin line of the target tooth of the 3D oral scan data and the region of interest may be manually operated by a user or be automatically operated by a deep learning. For example, the margin line of the target tooth of the 3D oral scan data and the region of interest may be determined using a second artificial intelligence neural network different from the first artificial intelligence neural network.

The first axis LR1, the second axis XA1 and the third axis BL1 of the 3D oral scan data in the region of interest may be determined based on the oral scan landmark.

For example, the first axis LR1 of the 3D oral scan data may be defined by a tangential direction of the teeth curve at the target tooth. The first axis LR1 of the 3D oral scan data may mean a horizontal direction of the target tooth.

For example, the second axis XA1 of the 3D oral scan data may represent an insertion direction of a tooth or an opposite direction of the insertion direction. For example, the second axis XA1 of the 3D oral scan data may mean a vertical direction of the target tooth. For example, the second axis XA1 may be perpendicular to the first axis LR1.

For example, the third axis BL1 of the 3D oral scan data may be perpendicular to the first axis LR1 and the second axis XA1, respectively. For example, the third axis BL1 of the 3D oral scan data may be defined by a cross product of a vector of the first axis LR1 and a vector of the second axis XA1.

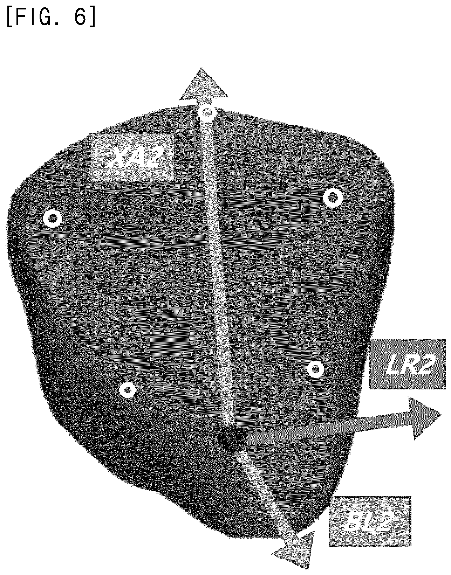

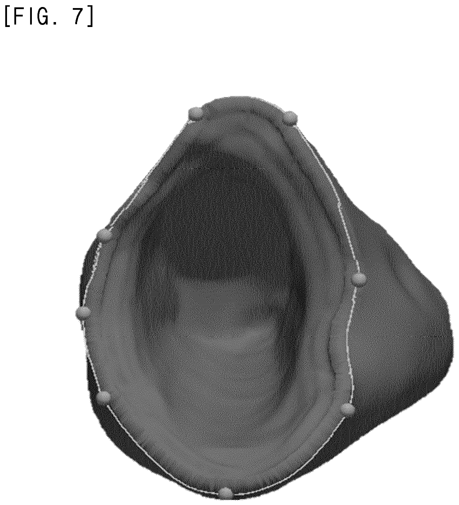

FIG. 6 is a diagram illustrating a fourth axis LR2, a fifth axis XA2 and a sixth axis BL2 of the 3D single tooth model of FIG. 1. FIG. 7 is a diagram illustrating a lower surface of the 3D single tooth model of FIG. 1 corresponding to the margin line of the 3D oral scan data of FIG. 1.

Referring to FIGS. 1 to 7, a single tooth landmark of the 3D single tooth model may be determined (step S400).

The step (step S400) of determining the single tooth landmark of the 3D single tooth model may be manually operated by a user or be automatically operated by a deep learning. For example, the single tooth landmark of the 3D single tooth model may be determined using a third artificial intelligence neural network different from the first artificial intelligence neural network and the second artificial intelligence neural network. Accordingly, in the automated method for aligning the 3D single tooth model to the 3D oral scan data of the present embodiment, three different artificial intelligence neural networks may be used.

The fourth axis LR2, the fifth axis XA2 and the sixth axis BL2 of the 3D single tooth model may be determined based on the single tooth landmark (step S500).

For example, the fourth axis LR2 of the 3D single tooth model may represent a horizontal direction of the tooth in the 3D single tooth model.

For example, the fifth axis XA2 of the 3D single tooth model may represent a direction toward an occlusal surface of the tooth in the 3D single tooth model or an opposite direction of the direction toward the occlusal surface. For example, the fifth axis XA2 may be perpendicular to the fourth axis LR2.

For example, the sixth axis BL2 of the 3D single tooth model may be perpendicular to the fourth axis LR2 and the fifth axis XA2, respectively. For example, the sixth axis BL2 of the 3D single tooth model may be defined by a cross product of a vector of the fourth axis LR2 and a vector of the fifth axis XA2.

For example, the single tooth landmark may include at least two landmarks defined on a tooth in the 3D single tooth model to determine the fourth axis LR2.

In addition, for example, the single tooth landmark may include at least two landmarks defined on the tooth in the 3D single tooth model to determine the fifth axis XA2.

In FIG. 6, examples of the single tooth landmarks are indicated with white circles.

The 3D single tooth model may be aligned to the 3D oral scan data such that the fourth axis LR2, the fifth axis XA2 and the sixth axis BL2 of the 3D single tooth model respectively overlap the first axis LR1, the second axis XA1 and the third axis BL1 of the 3D oral scan data (step S600).

Herein, the 3D single tooth model may be aligned to the 3D oral scan data such that an outline of the lower surface of the 3D single tooth model in FIG. 7 corresponds to the margin line of the target tooth in the 3D oral scan data.

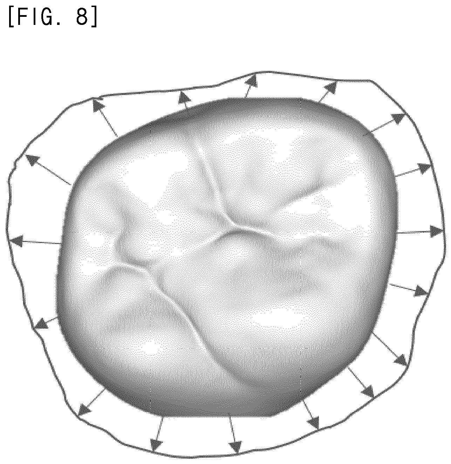

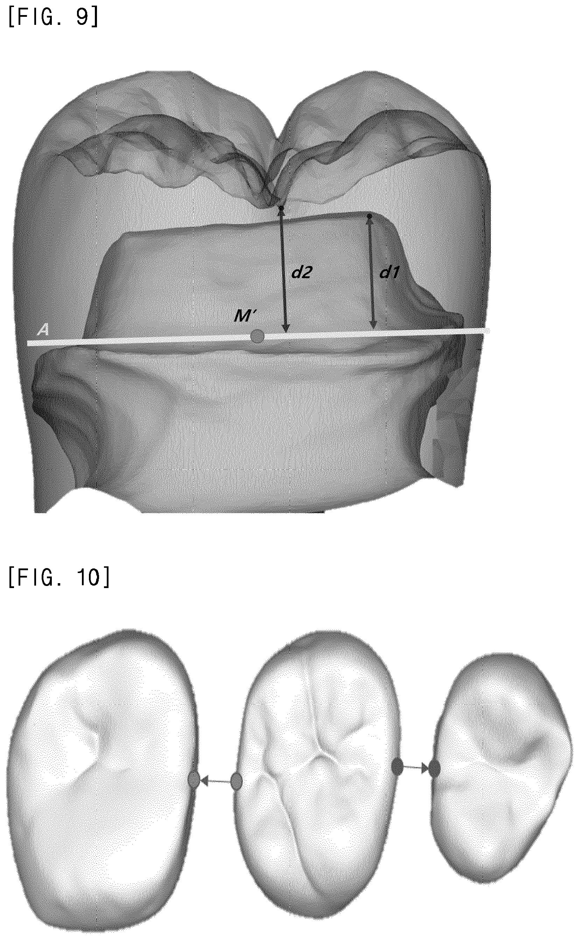

FIG. 8 is a diagram illustrating an example of scaling the 3D single tooth model of FIG. 1 based on the margin line of the 3D oral scan data of FIG. 1. FIGS. 9 and 10 are diagrams illustrating examples of scaling the 3D single tooth model of FIG. 1.

Referring to FIGS. 1 to 10, the 3D single tooth model may be scaled based on the margin line of the target tooth of the 3D oral scan data and the adjacent teeth adjacent to the target tooth (step S700).

For example, the 3D single tooth model may be scaled based on the margin line of the target tooth in the 3D oral scan data. When the result of initial alignment of the 3D single tooth model to the 3D oral scan data based on the coordinate axes is as shown in FIG. 8 (that is, a size of the 3D single tooth model is small than the margin line of the target tooth in the 3D oral scan data), the 3D single tooth model may be enlarged in the horizontal direction such that the 3D single tooth model corresponds to the margin line of the target tooth of the 3D oral scan data. In contrast, when the size of the 3D single tooth model is greater than the margin line of the target tooth in the 3D oral scan data, the 3D single tooth model may be reduced in the horizontal direction such that the 3D single tooth model corresponds to the margin line of the target tooth of the 3D oral scan data.

FIG. 9 represents an example of a method for adjusting a height of the 3D single tooth model. In FIG. 9, a point M' is a point in the prepared target tooth and a plane A is a plane defined in the 3D oral scan data by the point M' and the second axis XA. A first distance d1 represents a distance from the plane A to a furthest position of the prepared target tooth. A second distance d2 represents a distance from the plane A to a closest position of the occlusal surface of the 3D single tooth model. The height of the 3D single tooth model may be adjusted such that the second distance d2 matches the first distance d1.

In addition, the height of the 3D single tooth model may be adjusted based on heights of the adjacent teeth adjacent to the target tooth in the 3D oral scan data. For example, the height of the 3D single tooth model may be adjusted such that the height of the initially aligned 3D single tooth model matches the height of one of the adjacent teeth.

For example, as shown in FIG. 10, a width of the 3D single tooth model may be adjusted based on contact points where the 3D single tooth model contacts the adjacent teeth adjacent to the target tooth in the 3D oral scan data. When the initially aligned 3D single tooth model is spaced apart from the adjacent teeth, the width of the 3D single model may be enlarged to contact the adjacent teeth. In contrast, when the initially aligned 3D single tooth model is partially overlapped with the adjacent teeth, the width of the 3D single model may be reduced not to overlap the adjacent teeth.

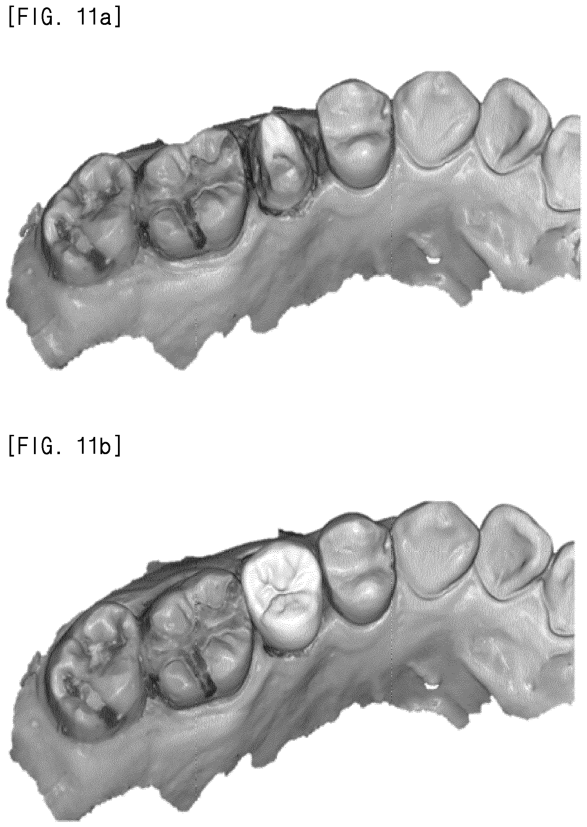

FIG. 11a is a diagram illustrating an example of 3D oral scan data and FIG. 11b is a diagram illustrating a result of combining a 3D single tooth model to the 3D oral scan data of FIG. 11a.

As shown in FIG. 11b, the 3D single tooth model may be aligned to the prepared tooth (the target tooth) in the 3D oral scan data of FIG. 11a. As explained above, the automated method for aligning the 3D single tooth model to the 3D oral scan data may include determining the oral scan landmark of the 3D oral scan data and the teeth curve formed by teeth in the 3D oral scan data (step S100), determining the margin line of the target tooth in the 3D oral scan data and the region of interest (step S200), determining the first axis, the second axis and the third axis of the 3D oral scan data in the region of interest based on the oral scan landmark (step S300), determining the single tooth landmark of the 3D single tooth model (step S400), determining the fourth axis, the fifth axis and the sixth axis of the 3D single tooth model based on the single tooth landmark (step S500) and aligning the 3D single tooth model to the 3D oral scan data such that the fourth axis, the fifth axis and the sixth axis of the 3D single tooth model respectively overlap the first axis, the second axis and the third axis of the 3D oral scan data (step S600).

In addition, the automated method for aligning the 3D single tooth model to the 3D oral scan data may further include scaling the 3D single tooth model based on the margin line of the target tooth and adjacent teeth adjacent to the target tooth of the 3D oral scan data (step S700) after the initial alignment.

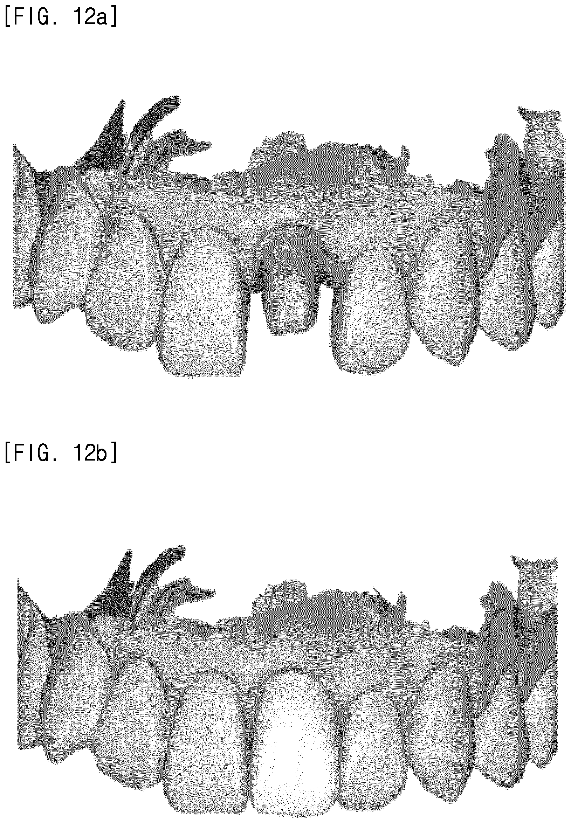

FIG. 12a is a diagram illustrating an example of 3D oral scan data and FIG. 12b is a diagram illustrating a result of combining a 3D single tooth model to the 3D oral scan data of FIG. 12a.

As shown in FIG. 12b, the 3D single tooth model may be aligned to the prepared tooth (the target tooth) in the 3D oral scan data of FIG. 12a.

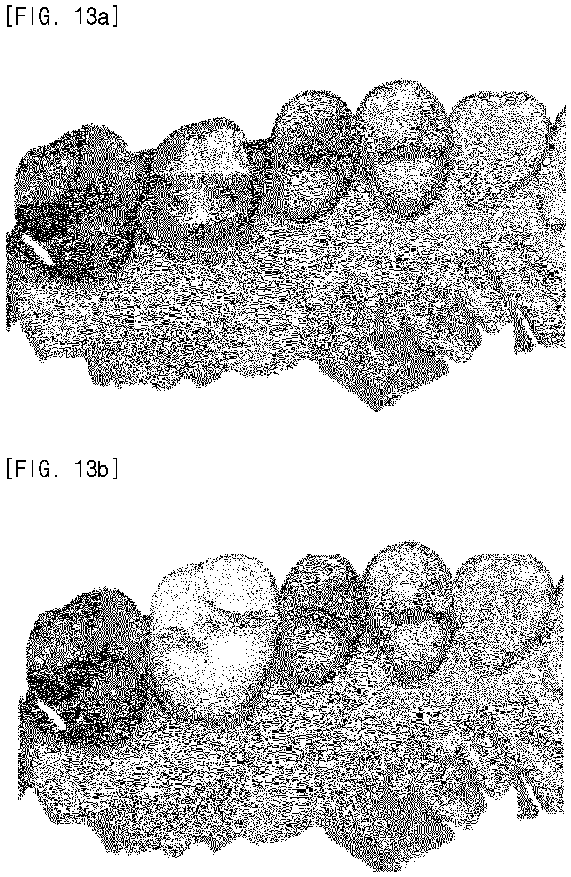

FIG. 13a is a diagram illustrating an example of 3D oral scan data and FIG. 13b is a diagram illustrating a result of combining a 3D single tooth model to the 3D oral scan data of FIG. 13a.

As shown in FIG. 13b, the 3D single tooth model may be aligned to the prepared tooth (the target tooth) in the 3D oral scan data of FIG. 13a.

According to the present embodiment, the process of aligning the 3D single tooth model to the 3D oral scan data is performed automatically so that a work fatigue of the dentist or dental technician may decrease and an accuracy of the aligning result may increase.

In addition, the aligned single tooth model may be used to manufacture prostheses, implants, braces and dental instruments so that an effort and a time of manufacturing the prostheses, the implants, the braces and the dental instruments may decrease and an accuracy and a productivity of the prostheses, the implants, the braces and the dental instruments may increase.

In addition, a deep learning may be used in some steps of the automated method for aligning the 3D single tooth model to the 3D oral scan data. When the deep learning is be used in some steps, the work fatigue of the dentist or dental technician may further decrease and the accuracy of the aligning result may further increase.

According to an embodiment of the present inventive concept, a non-transitory computer-readable storage medium having stored thereon program instructions of the automated method for aligning the 3D dental library model to the 3D oral scan data may be provided. The above-mentioned method may be written as a program executed on the computer. The method may be implemented in a general purpose digital computer which operates the program using a computer-readable medium. In addition, the structure of the data used in the above mentioned method may be written on a computer readable medium through various means. The computer readable medium may include program instructions, data files and data structures alone or in combination. The program instructions written on the medium may be specially designed and configured for the present inventive concept, or may be generally known to a person skilled in the computer software field. For example, the computer readable medium may include a magnetic medium such as a hard disk, a floppy disk and a magnetic tape, an optical recording medium such as CD-ROM and DVD, a magneto-optical medium such as floptic disc and a hardware device specially configured to store and execute the program instructions such as ROM, RAM and a flash memory. For example, the program instructions may include a machine language codes produced by a compiler and high-level language codes which may be executed by a computer using an interpreter or the like. The hardware device may be configured to operate as one or more software modules to perform the operations of the present inventive concept.

In addition, the above mentioned automated method for aligning the 3D single tooth model to the 3D oral scan data may be implemented in a form of a computer-executed computer program or an application which are stored in a storage method.

### [INDUSTRIAL AVAILABILITY]

The present inventive concept is related to the automated method for aligning the 3D single tooth model to the 3D oral scan data and the non-transitory computer-readable storage medium having stored thereon program instructions of the automated method for aligning the 3D single tooth model to the 3D oral scan data. According to the present inventive concept, an effort and a time of manufacturing the prostheses, the implants, the braces and the dental instruments may decrease and an accuracy and a productivity of the prostheses, the implants, the braces and the dental instruments may increase.

Although a few embodiments of the present inventive concept have been described, those skilled in the art will readily appreciate that many modifications are possible in the embodiments without materially departing from the novel teachings and advantages of the present inventive concept. Accordingly, all such modifications are intended to be included within the scope of the present inventive concept as defined in the claims.

## Claims

1. An automated method for aligning a three dimensional ("3D") single tooth model to 3D oral scan data, the method comprising:
determining an oral scan landmark of the 3D oral scan data and a teeth curve formed by teeth in the 3D oral scan data;
determining a margin line of a target tooth in the 3D oral scan data and a region of interest in the 3D oral scan data;
determining a first axis, a second axis and a third axis of the 3D oral scan data in the region of interest based on the oral scan landmark;
determining a single tooth landmark of the 3D single tooth model;
determining a fourth axis, a fifth axis and a sixth axis of the 3D single tooth model based on the single tooth landmark; and
aligning the 3D single tooth model to the 3D oral scan data such that the fourth axis, the fifth axis and the sixth axis of the 3D single tooth model respectively overlap the first axis, the second axis and the third axis of the 3D oral scan data.

2. The method of claim 1, further comprising scaling the 3D single tooth model based on the margin line of the target tooth and adjacent teeth adjacent to the target tooth of the 3D oral scan data.

3. The method of claim 2, further comprising adjusting a height of the 3D single tooth model based on a height of an adjacent tooth adjacent to the target tooth in the 3D oral scan data.

4. The method of claim 2, further comprising adjusting a width of the 3D single tooth model based on a contact point where the 3D single tooth model contacts an adjacent tooth adjacent to the target tooth in the 3D oral scan data.

5. The method of claim 1, wherein the first axis of the 3D oral scan data is defined by a tangential direction of the teeth curve at the target tooth.

6. The method of claim 5, wherein the second axis of the 3D oral scan data represents an insertion direction of a tooth or an opposite direction of the insertion direction.

7. The method of claim 6, wherein the third axis of the 3D oral scan data is defined by a cross product of a first vector of the first axis and a second vector of the second axis.

8. The method of claim 5, wherein the oral scan landmark includes at least three landmarks disposed in the 3D oral scan data.

9. The method of claim 8, wherein the oral scan landmark includes a first landmark disposed at a first end portion of the teeth curve, a second landmark disposed at a second end portion of the teeth curve and a third landmark disposed at a central point of the teeth curve.

10. The method of claim 1, wherein the fourth axis of the 3D single tooth model represents a horizontal direction of the tooth in the 3D single tooth model.

11. The method of claim 10, wherein the fifth axis of the 3D single tooth model represents a direction toward an occlusal surface of the tooth in the 3D single tooth model or an opposite direction of the direction toward the occlusal surface.

12. The method of claim 11, wherein the sixth axis of the 3D single tooth model is defined by a cross product of a fourth vector of the fourth axis and a fifth vector of the fifth axis.

13. The method of claim 11, wherein the single tooth landmark includes at least two landmarks defined on a tooth in the 3D single tooth model to determine the fourth axis.

14. The method of claim 13, wherein the single tooth landmark includes at least two landmarks defined on the tooth in the 3D single tooth model to determine the fifth axis.

15. The method of claim 1, wherein the oral scan landmark and the teeth curve are determined using a first artificial intelligence neural network.

16. The method of claim 15, wherein the margin line of the target tooth in the 3D oral scan data and the region of interest are determined using a second artificial intelligence neural network different from the first artificial intelligence neural network.

17. The method of claim 16, wherein the single tooth landmark of the 3D single tooth model is determined using a third artificial intelligence neural network different from the first artificial intelligence neural network and the second artificial intelligence neural network.

18. The method of claim 1, further comprising adjusting a height of the 3D single tooth model based on a first distance from a first plane in the 3D oral scan data to a first point of the target tooth in the 3D oral scan data and a second distance from the first plane to a second point of the 3D single tooth model.

19. The method of claim 18, wherein the first distance represents a distance from the first plane to a furthest position of the target tooth,
wherein the second distance represents a distance from the first plane to a closest position of an occlusal surface of the 3D single tooth model, and
wherein the height of the 3D single tooth model is adjusted such that the second distance matches the first distance.

20. A non-transitory computer-readable storage medium having stored thereon at least one program comprising commands, which when executed by at least one hardware processor, perform the method of claim 1.
